# EUROPEAN PATENT SPECIFICATION

(11) **EP 1 554 012 B1**
(45) Date of publication and mention of the grant of the patent: **01.08.2007**
(21) Application number: 03770411.1
(22) Date of filing: 25.09.2003
(51) Int. Cl.: A61N 1/372, G08C 17/02, G08C 19/00, H04B 1/38, H04B 1/40, H04Q 7/38

(54) **MULTI-MODE PROGRAMMER FOR MEDICAL DEVICE COMMUNICATION**
MULTIMODALE PROGRAMMIER-EINRICHTUNG ZUR DATENÜBERMITTLUNG VON MEDIZINISCHEM GERÄT
PROGRAMMATEUR MULTIMODE DESTINE A UNE COMMUNICATION AVEC UN DISPOSITIF MEDICAL

(30) Priority: 30.09.2002 US 261317
(43) Date of publication of application: 20.07.2005
(73) Proprietor: MEDTRONIC, INC., Minneapolis, MN 55432 (US)
(72) Inventor: THOMPSON, David, L., Andover, MN 55304 (US); GREENINGER, Daniel, R., Coon Rapids, MN 55448 (US); WEIJAND, Koen, J., NL-3223 Hellevoetsluis (NL)
(74) Representative: Hughes, Andrea Michelle
(86) International application number: PCT/US2003/030109
(87) International publication number: WO 2004/030757

(56) References cited:
- US-A- 5 752 976
- US-A- 6 083 248
- US-A- 6 167 312
- US-B1- 6 169 925
- US-B1- 6 292 698

## Description

The invention relates to medical devices and, more particularly, medical devices equipped to communicate via telemetry. '

### BACKGROUND

Telemetry generally refers to communication of data, instructions, and the like between a medical device and a medical de vice programmer. For example, the programmer may use telemetry to program a medical device to deliver a particular therapy to a patient. In addition, the programmer may use telemetry to interrogate the medical device. In particular, the programmer may obtain diagnostic data, event marker data, activity data and other data collected or identified by the medical device. The data may be used to program the medical device for delivery of new or modified therapies. In this manner, telemetry between a medical device and a programmer can be used to improve or enhance medical device therapy.

Telemetry typically involves wireless data transfer between a medical device and the programmer using radio frequency (RF) signals, infrared (IR) frequency signals, or other electromagnetic signals. Any of a variety of modulation techniques may be used to modulate data on a respective electromagnetic carrier wave. Alternatively, telemetry may be performed using wired connections, sound waves, or even the patient's flesh as the transmission medium. A number of different telemetry systems and techniques have been developed to facilitate the transfer of data between a medical device and the associated programmer.

Many implantable medical devices (IMDs) support telemetry. Examples of an IMD include implantable cardiac pacemakers, implantable defibrillators, implantable pacemaker/cardioverter/defibrillators, implantable muscular stimulus devices, implantable brain stimulators, other implantable organ stimulation devices, implantable drug delivery devices, implantable monitors, and the like. Telemetry, however, is not limited to communication with IMDs. For example, telemetry may also be used to communicate with non-implanted medical devices in substantially the same way as it is used with DADS.

The evolution and advancement of telemetry has yielded a number of advances in the art including, for example, improved communication integrity, improved data transmission rates, improved communication security, and the like. Moreover, as new therapeutic techniques are developed, telemetry allows the new techniques to be programmed into older medical devices, including devices previously implanted in a patient. Unfortunately, the evolution of telemetry has also resulted in proliferation of a wide variety of different systems and communication techniques that generally require a unique programmer for communication with each type of device. Consequently, ' different types of medical devices, medical devices manufactured by di$erent companies, or even similar medical devices manufactured by the same company, often employ different telemetry techniques. Accordingly, a wide variety of different programmers are needed to communicate with different medical devices in accordance with the different telemetry techniques employed by the medical devices.

### SUMMARY

In general, the invention is directed to a multi-mode programmer for communication with different medical devices that utilize different telemetry communication techniques. The programmer receives telemetry signals from a given medical device, and selects an appropriate communication mode, which can be pre-programmed into the programmer as one of a plurality of possible communication modes. For example, upon receiving a telemetry signal from the medial device, the programmer may identify a signature associated with the received telemetry signal. The programmer can then select the appropriate communication mode, such as by accessing a lookup table that associates signatures with communication modes. Accordingly, the programmer can selectively configure itself for communication with a given medical device based on the telemetry signal it receives from that medical device.

In one aspect, the invention provides a method for providing communication between a programmer and a plurality of different medical devices which communicate with said programmer using respective, different types of telemetry techniques; said method comprising: providing a programmer having a plurality of possible types of telemetry communication techniques stored in a memory thereof, receiving at a control unit of said programmer a first signal from a medical device; and selecting by means of said control unit a type of communication technique from said plurality of possible communication techniques based on the first signal, for communicating with said medical device.

For example, selecting the communication technique based on the first signal may include identifying a signature that substantially correlates to the first signal, and selecting a communication technique associated with the signature.

In another aspect, the invention provides a programmer for a plurality of different medical devices which communicate with the programmer using different telemetry techniques, said programmer comprising:
memory storing a plurality of possible telemetry communication techniques for the programmer; and
a control unit to receive a first signal from a medical device (8) and select one of the communication techniques based on the first signal to communication with said medical device.

In another embodiment, the invention provides a computer-readable medium comprising instructions that cause a medical device programmer to select a communication mode from a plurality of possible communication modes based on a first signal received from a medical device.

In another embodiment, the invention provides a system comprising a first medical device, a second medical device, and a programmer. For example, the programmer receives a first signal from the first medical device, selects a first communication mode from a plurality of possible communication modes based on the first signal, generates a second signal that complies with the first communication mode, sends the second signal to the first medical device, receives a third signal from the second medial device, selects a second communication mode from the plurality of possible communication modes based on the third signal, generates a fourth signal that complies with the second communication mode, and sends the fourth signal to the second medical device.

The invention provides various advances in the art. In particular, the invention can eliminate the need for multiple programmers for telemetric communication with different medical devices. Instead, a multi-mode programmer can be used to communicate with a plurality of different medical devices on a selective basis. In addition, the invention may find useful application as an interrogator in emergency , (first responder and emergency room) scenarios by facilitating the ability to identify and communicate with medical devices used by a given patient. In that case, the ability to obtain diagnostic and therapeutic information from a given medical device without requiring knowledge of the make and model of the device may save valuable time, possibly saving lives. In this embodiment, the programming of parameters may not be available, which may further reduce device size and complexity. The invention may also provide distinct advances in the art in terms of the size (form factor) and mechanical configuration of a programmer, useful for patient activation/control. For example, a number of mechanical configurations are envisioned, including wearable configurations such as configurations similar to jewelry, a wrist watch or a belt buckle to be worn by the patient or medical personnel. In addition, a programmer in the form of an ID card or adhesive patch with a removable memory card are envisioned for use by a patient so that information can be collected on the removable memory card when the patch is adhered to the patients skin. In that case, the memory card may be removed from the programmer and sent to a physician for analysis without the need to send the entire programmer to the physician. Accordingly, the programmer can be reused with another memory card. Of course, the programmer itself could also be sent by the patient to the physician, in accordance with other embodiments. These and other unique wearable configurations can be realized in various embodiments of the invention, some of which may have dimensions less than approximately 60 millimeters by 90 millimeters by 1 5 millimeters, i.e., a form factor similar to that of a thick credit card.

The techniques described herein may be implemented in a programmer in hardware, software, firmware, or any combination thereof. If implemented in software, invention may be directed to a computer readable medium comprising program code, that when executed, performs one or more of the techniques described herein. Additional details of various embodiments are set forth in the accompanying drawings and the description below. Other features, objects and advances in the art will become apparent from the description and drawings, and from the claims.

### BRIEF DESCRIPTION OF DRAWINGS

FIG. 1 is a conceptual diagram illustrating a multi-mode programmer communicating with an exemplary medical device implanted in a human body.
FIG. 2 is a block diagram illustrating telemetry between a multi-mode programmer and a set of different medical devices.
FIG. 3 is a block diagram of a multi-mode programmer that supports a plurality of communication modes for communicating with different medical devices via different telemetry techniques.
FIG. 4 is a more detailed block diagram of a multi-mode programmer according to an embodiment of the invention.
FIGS. 5 and 6 are diagrams illustrating an embodiment of a multi-mode programmer taking the form of an adhesive patch.
FIGS. 7 and 8 are flow diagrams illustrating techniques in accordance with embodiments of the invention.

### DETAILED DESCRIPTION

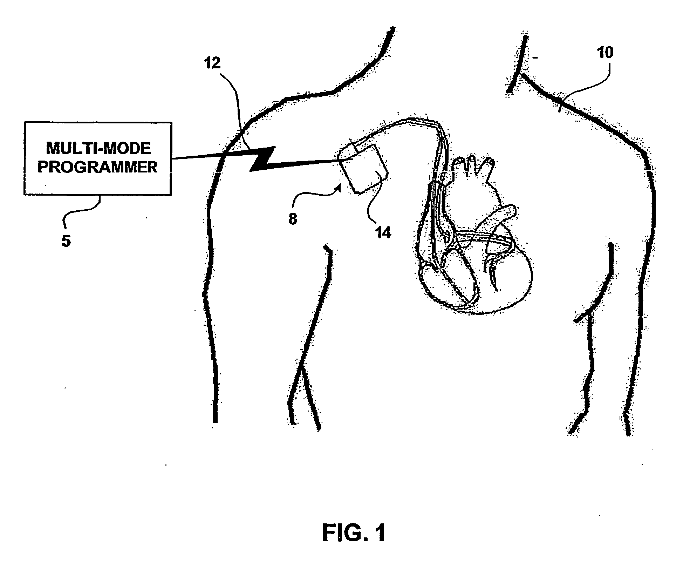

FIG. 1 is a conceptual diagram illustrating a multi-mode programmer 5 communicating with an exemplary medical device δ implanted in a human body 10. Medical device 8 represents one of a variety of medical devices that may communicate with programmer 5. Although illustrated as an implantable cardiac pacemaker, medical device 8 may take the form of a variety of other medical devices such as, for example, an implantable defibrillator, an implantable pacemaker/cardioverter/defibrillator, an implantable muscular stimulus device, an implantable brain stimulator, an implantable nerve stimulator, an implantable drug delivery device, implantable monitor, or the like. In addition, medial device 8, as described herein, is not necessarily limited to an implantable device. Also, in some cases, medical device 8 may correspond to a medical device used on non-human mammals or other animals. In short, the techniques described herein may be readily used with a wide variety of medical devices including implanted and non-implanted medical devices used to deliver therapy or perform diagnosis in humans, mammals, or other types of living beings.

In the example shown in FIG. 1, medical device 8 includes a hermetically-sealed enclosure 14 that may include various elements, although the invention is not limited to hermetically sealed devices. By way of example, enclosure 14 may house an electrochemical cell, e.g., a lithium battery, circuitry that controls device operations and records sensed events, physiogical activity and patient conditions, and a control unit coupled to an antenna to transmit and receive information via wireless telemetry signals 12.

Programmer 5 communicates with medical device 8 via telemetry signals 12. For example, programmer 5 may use telemetry signals 12 to program medical device 8 to deliver a particular therapy to human body 10, such as electrical stimulation, drug administration or the like. In addition, medical device 8 may use telemetry signals 12 to send information to programmer 5 such as diagnostic information, sensed conditions associated with the patient, information relating to therapy delivered to the patient, or any other information collected or identified by medical device 8. In this manner, telemetry allows communication between medical device 8 and programmer 5. In accordance with the invention, programmer 5 supports communication via a number of different telemetry modes. Accordingly, programmer 5 is capable of receiving and interpreting telemetry signals sent by medical devices that use different types of telemetry. Moreover, programmer 5 can communicate to different medical devices using selected communication modes that correspond to the given medical device with which programmer 5 is currently communicating. The different telemetry modes of programmer 5 may cause programmer 5 to select different telemetry techniques. For example, programmer 5 may be equipped to detect characteristic features of signals sent to programmer 5 via different communication modes, such as unique carrier waveform shapes, amplitudes, frequency and/or timing of the modulated waveform, or the like. Based on the detected characteristics, programmer 5 selects one of the telemetry modes appropriate for communication with medical device 8. Programmer 5 may be embodied in a wide variety of mechanical configurations. By way of example, programmer 5 may comprise a device worn on a patient's wrist, much like a wrist watch, and may even comprise a fully functional wrist watch that tells time, but also includes the programmer functionality described herein. Alternatively, programmer 5 may be worn around a patients neck, like a necklace, or around a patients waist, like a belt. In other configurations, programmer 5 may be embodied in an identification card, a pendent, a laptop computer, a handheld computer, a pager, or the like. In some cases, programmer 5 may comprise a programmed computer used by emergency medical personnel, e.g., in an ambulance, to communicate with a variety of possible medical devices that may be implanted within a given patient. In still other cases, programmer 5 may be embodied as an adhesive patch that is adhered to a patient's skin. These and other configurations of programmer 5 may be used in accordance with the invention.

In any case, programmer 5 receives telemetry signals 12 from a given medical device 8, and dynamically selects an appropriate communication mode, which can be pre-programmed into programmer 5 as one of a plurality of possible communication modes. For example, upon receiving a telemetry signal 12 from medical device 8, programmer 5 may identify a signature associated with the telemetry signal 12. Programmer 5 may then select the appropriate communication mode, such as by accessing a lookup table (LUT) that associates signatures with communication modes. Then, the programmer 5 can configure itself for communication with medical device 8 based on the telemetry signal 12 received from medical device 8.

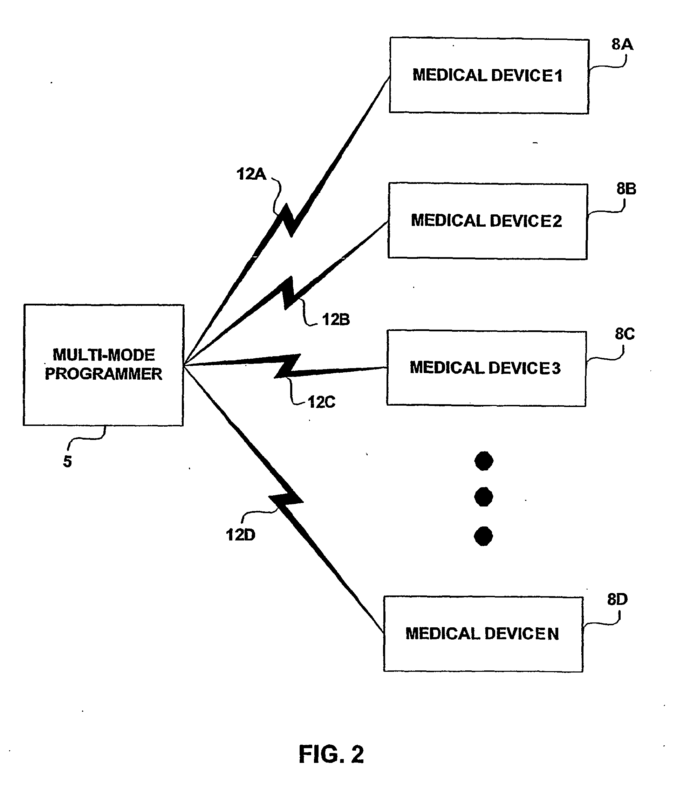

FIG. 2 is a block diagram illustrating telemetry between programmer 5 and a set of different medical devices 8A-8D. Again, the different medical devices 8A-8D may comprise any of a wide variety of medical devices, including implanted and non-implanted medical devices, used to deliver therapy to humans, mammals, or even other types of living beings. In accordance with the invention, the different devices 8A-8D communicate using different telemetry techniques. In other words, the format of telemetry signal 12A is different from that of 12B, 12C and 12D. For example, different telemetry signals 12 may have distinct carrier waveforms defined by amplitude and frequency. Also, different telemetry signals 12 may be modulated differently, e.g., using amplitude modulation (AM), frequency modulation (FM), pulse width modulation (PWM), pulse code modulation (PCM), pulse position modulation (PPM), or the like. Also, different coding schemes may be associated with different signals 12, such as phase-shift keying (PSK), orthogonal coding, frame based coding, or the like. Programmer 5 may identify these unique characteristics of the raw signal without performing a demodulation in order to identify the communication mode. An appropriate demodulator can then be selected, as well as appropriate signal transmission techniques and components.

Programmer 5 supports communication with the different devices 8A-8D by supporting communication via each of the different telemetry communication modes associated with signals 12A-12D. In particular, programmer 5 selectively switches communication modes to match the communication mode of medical device 8, and thereby permit programming, interrogation or both. Programmer 5 may be configured to receive signals in a frequency band known to correlate to all of telemetry signals 12, or may periodically tune to different frequency bands to tune for reception of different telemetry signals 12 over time.

The different medical devices 8A-8D may correspond to different types of devices, i.e., devices that deliver different types of therapy. Alternatively medical devices 8A-8D may comprise similar devices manufactured by different companies, which use different telemetry techniques. In addition, medical devices 8A-8D may correspond to similar devices manufactured by the same company, but which use different telemetry techniques.

In most cases, medical devices 8A-8D correspond to different devices implanted or used on different patients. In some cases, however, medical devices 8A-8D may correspond to different devices implanted or used in one particular patient. In other words, a patient may have more than one medical device 8 implanted within his or her body. In that case, programmer 5 may support communication with all of the different devices implanted and used within the same patient. In any case, the need for distinct programmers for each device can be eliminated in favor of a single multi-mode programmer 5 that supports a plurality of communication modes.

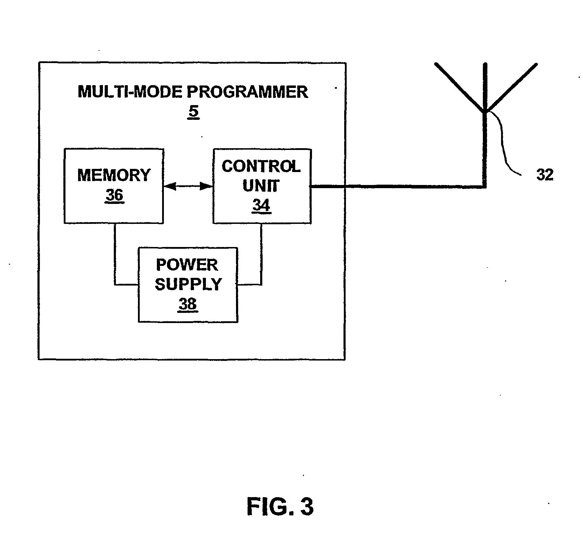

FIG. 3 is an exemplary block diagram of a programmer 5 that supports a plurality of communication modes for communicating to different medical devices via different telemetry techniques. Programmer 5 is configured to dynamically select different communication modes according to the communication modes presented from medical devices 8. As illustrated, programmer 5 may include an antenna 32, a control unit 34, a memory 36, and a power supply 38.

Antenna 32 may send and receive different electromagnetic telemetry signals 12, such as radio frequency signals, as directed by control unit 34. The invention, however, is not limited for use with electromagnetic telemetry signals, but may also used with other telemetry signals, including sound waves. In addition, in some embodiments, programmer 5 may use the patients flesh as a transmission line for communication of electromagnetic signals between medical devices and programmer 5.

In any case, programmer 5 supports communication according to a plurality of telemetry modes. In operation, control unit 34 of programmer 5 receives telemetry signals via antenna 32. Antenna 32 may be tuned to a large frequency band that covers any possible telemetry signal that may be received from a device supported by programmer 5, or may be periodically tuned by control unit 34 to individual frequencies that correspond to specific telemetry signals that are supported. In any case, once a signal is received, control unit 34 conditions received signals so that signatures associated with the received signals can be identified. For example, control unit 34 may perform amplification or attenuation on received signals, and may also implement a phase locked loop to properly synchronize the phase of a received signal with the signatures to which the received signal is being compared.

The signatures may correspond to templates of expected waveforms that correspond to possible telemetry signals that could be received. The signatures may include distinctive waveform characteristics indicative of the respective telemetry signal, such as a particular frequency, amplitude, shape, modulation characteristic, or the like. Memory 36 stores the signatures for every telemetry technique that is supported by programmer 5. Accordingly, control unit 34 compares received signals with stored signatures by accessing memory 36. Then, after identifying an acceptable match between a stored signature and a received telemetry signal, e.g., 12A, programmer 5 is able to identify the telemetry technique associated with the medical device that sent signal 12A. In other words, the received signals can be compared to signatures, and the signatures can be mapped to communication modes.

Memory 36 may also store configuration parameters associated with different communication modes for control unit 34. In addition, memory 36 may include a lookup table (LUT) that maps signatures to communication modes, i.e., by mapping a number associated with a signature to a number associated with an associated communication mode. Thus, upon identifying a signature associated with a received telemetry signal 12A, control unit can access the LUT in memory 36 to select the proper communication mode. Then, control unit 34 can be configured according to the selected communication mode to output telemetry signals that the medical device associated with the received telemetry signal 12A can understand. In addition, control unit 34 can configure itself so that signals sent from the respective device can be properly demodulated and interpreted. In short, the different communication modes supported by programmer 5 can be programmed into memory 36, and then applied on a selective basis based on received telemetry signals 12.

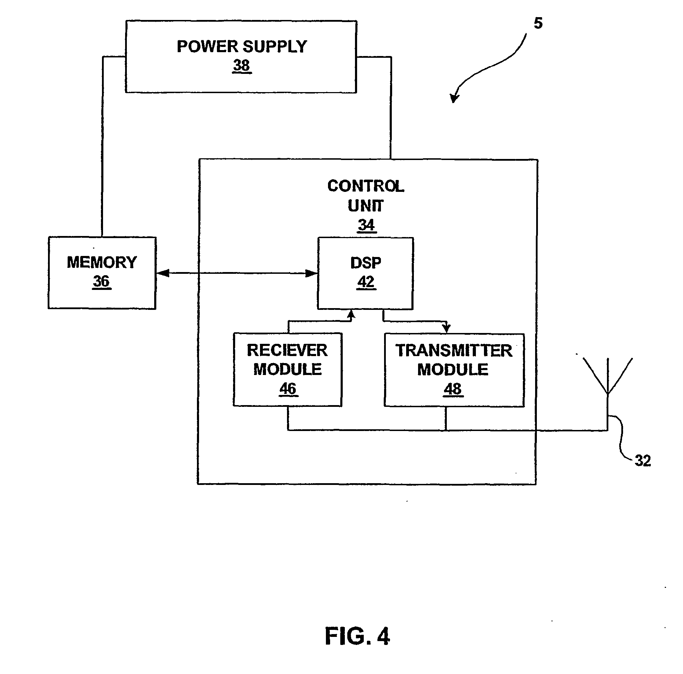

FIG. 4 is a more detailed exemplary block diagram of programmer 5. As illustrated, programmer 5 includes a power supply 38, such as a battery, that powers control unit 34 and memory 36. Antenna 32 is coupled to control unit 34 to facilitate the reception and transmission of wireless electromagnetic telemetry signals. The invention, however, is not necessarily limited for use with wireless signals or electromagnetic signals. Again, similar principles can be applied in a programmer that can be wired to one or more medical devices, or a programmer that uses the patient's flesh or sound waves as the transmission medium for telemetric communication. Control unit 34 may include a programmable digital signal processor (DSP) 42 coupled to a crystal oscillator (not shown). Examples of suitable DSPs include the TI-TMS320C2000 family of DSPs, such as the model number TI-TMS320LC2406 DSP, commercially available from Texas Instruments Incorporated of Dallas Texas, USA. By way of example, the oscillator may comprise a 5 MHz crystal, although other oscillators could be used. The TI-TMS320LC2406 DSP is a 16-bit fixed point DSP originally designed for motor control applications. The TI-TMS320LC2406 DSP includes internal flash memory and a 10-bit analog to digital converter (ADC). Other DSPs and programmable microprocessors, however, could alternatively be used. Memory 36 may comprise a removable memory card that couples to DSP 42 via a memory connector, although non-removable memory could also be used. Removable memory cards can provide an added benefit in that the card can be removed from programmer 5 and sent to a physician for analysis. For example, after programmer 5 telemetrically communicates with a given medical device 8, data from that medical device may be stored in memory 36. The data stored in memory 36 may be data selected by programmer 5. In some cases, the data stored in memory 36 may be overflow data from an internal memory associated with medical device 8, allowing programmer 5 to provide more continuous and more prolonged patient monitoring capabilities. If memory 36 comprises a removable card, the card may be removed from programmer 5 and sent to a physician, and a new card may be inserted in its place. In this manner, data from a medical device 8 can be easily provided to a physician, e.g., to facilitate early diagnosis of problems.

Moreover, the use of memory cards can avoid the need to send the whole programmer 5 to the physician. In addition, a more continuous and larger sample of data from the medial device may be captured by sequentially inserting a number of memory cards into programmer 5 over a period of time in which information is being sent from the respective medical device. As one example, memory 36 may comprise a 64 or 256 Megabyte multimedia memory module commercially available by SanDisk of Sunnyvale, California, USA. Other removable or non-removable memory, however, may also be used.

Another advances in the art of removable memory cards and a DSP relates to updating the function of the programmer 5. For example, in order to update programmer 5 to support new or different forms of telemetric communication, a different memory card, storing software to support the new or different telemetry may be provided. In other words, a DSP configuration with removable memory provides advances in the art in terms of scalability of programmer 5. If new or different telemetry is developed, software can be likewise devolved and provided to programmer via a new removable memory card. Accordingly, in that case, the need to develop a different programmer may be avoided. Instead, new algorithms can be provided to programmer 5 via a new memory card that stores new instructions that can be executed by the DSP.

Antenna 32 may comprise any of a wide variety of antenna configurations. In one particular example, antenna 32 may comprise a substantially flat, co-planer dual opposing coil antenna. For example, two opposing coils may be formed on a common substrate to provide two signal inputs to control unit 34. The input of two or more signals to control unit 34 may simplify signal processing within control unit 34, such as by simplifying filtering. In addition, an antenna scheme utilizing multiple concentric and co-planar antenna coils on a substrate may also reduce the form factor of programmer 5, which can facilitate wearable embodiments. The use of concentric and co-planar antenna coils may also improve the reception of telemetry signals in a noisy environment.

Power supply 38 may comprise any of a wide variety of batteries or other power sources. For example, power supply 38 may comprise a rechargeable or non-rechargeable battery, such as a polymer or foil battery, a lithium ion batter, a nickel cadmium battery, or the like. The battery may have a voltage range of approximately 4.2 to 3.0 volts throughout its useful service life and a capacity of 1.5 Ah, although the invention is not limited in that respect.

In addition to DSP 42, control unit 34 of programmer 5 may include a receiver module 46 and a transmitter module 48. Receiver module 46 and transmitter module 48 may be integrated or may comprise separate circuits. The composition of receiver module 46 and transmitter module 48 may depend on the particular DSP 42 used in control unit 34 as well as the particular communication modes supported by programmer 5.

In general, receiver module 46 conditions a received telemetry signal for analysis by DSP 42. Receiver module 46 may include an analog-to-digital converter (ADC), although some DSPs, such as the TI-TMS320LC2406 mentioned above, include an ADC as part of the DSP. Receiver module 46 may also include one or more amplifiers, a variable gain amplifier (VGA), one or more filters, automatic gain control (AGC), if needed, and a phase-locked loop for synchronizing a received signal so that an in-phase sample can be identified. These and/or other components of receiver module 46 condition a received telemetry signal as required by DSP 42 so that signal analysis can be performed. In some cases, DSP 42 may configure both itself and receiver module 46 for reception of a given telemetry signal that is expected, such as by selectively switching on a subset of the bandpass filters in DSP 42 and controlling the gain of a received signal in receiver module 46.

Transmitter module 48 conditions output signals for wireless transmission to a medical device via antenna 38. For example, DSP 42 may generate timed output signals based on a selected communication mode in order to communicate with the respective medical device 8 via telemetry. Transmitter module 48 can receive signals from DSP 42 and amplify the signals for transmission via antenna 38. For example, transmitter module 48 may include transmit circuitry for driving antenna 38, such as a set of field effect transistors (FET) that output relatively large output voltage pulses in response to relatively small input voltages received from DSP 42. Transmitter module 48 may also include various other filters, amplifiers, or the like, that may be selectively activated based on the given communication mode. For example, in some cases, a selected communication mode identified by DSP 42 can cause DSP 42 to send control signals to transmitter module 48 to configure transmitter module 48 for telemetric communication consistent with the selected communication mode. In any case, transmitter module 48 conditions output signals from DSP 42 for wireless telemetric transmission to a medical device.

DSP 42 of programmer 5 may include several different bandpass filters and several different demodulators, such as one or more amplitude demodulators, one or more frequency-shift keyed (FSK) demodulators, one or more phase-shift keyed (PSK) demodulators, and the like. For example, these different components may be programmed as software or firmware. In any case, DSP 42 selects the particular bandpass filter(s) and demodulator type to process the digitized signal according to the communication mode that is selected. In other words, DSP compares the raw signal that is received to signatures in order to identify the appropriate communication mode, and then selectively enables the appropriate demodulator so that subsequent signals can be demodulated and interpreted.

An additional function implemented by DSP 42 may include the control of a variable-gain amplifier (VGA) or other components included in receiver module 46 or transmitter module 48. For example, this may further ensure that the receiver module 46 supplies to the A/D converter of DSP 42 a signal having a desired peak amplitude. Moreover, VGA control in the DSP 42 may provide flexibility in software so that adjustments can be made to properly condition a wide variety of telemetry signals. In order to facilitate the automatic gain control (AGC) between DSP 42 and receiver module 46, receiver module 46 may include a digital-to-analog (D/A) converter to convert a digital control word supplied from DSP 42 to a corresponding analog voltage level for variable-gain amplification.

One specific configuration of programmer 5 may be formed of the exemplary components listed above including the TI-TMS320LC2406 DSP, SanDisk memory module, a dual coil planer antenna, a sufficiently small battery, and individual hardware components to implement the receiver module 46 and transmitter module 48. In that case, programmer 5 may realize a compact form factor suitable for inconspicuous use by a patient, e.g., to collect information from a medical device and send the memory cards to the physician. A minimal amount of communication from programmer 5 to the medical device may prompt the medical device to uplink the requested information. For example, such exemplary components may be used to realize a programmer 5 having dimensions less than approximately 60 millimeters by 90 millimeters by 15 millimeters. In other words, programmer 5 can be made to dimensions corresponding roughly to the size of a thick credit card. Such reduced size can be particularly useful for wearable embodiments of programmer 5.

If desired, programmer 5 may also include an activation switch (not shown), to allow a patient to initiate communication with a medical device. For example, if the patient identifies pain or other problems, it may be desirable to initiate communication, e.g., to cause the medical device to communicate sensed information to programmer 5. In that case, an activation switch can provide the patient with the ability to ensure that sensed conditions are stored in programmer 5 during periods of time when physical problems may be occurring to the patient.

Moreover, programmer 5 may include other user interface features, such as a display screen, a speaker, or a blinking light. For example, feedback in the form of sound or light flashes, images, instructions, or the like may be useful to a patient, e.g., to indicate that communication has been initiated or to indicate to the patient that the programmer is positioned correctly for such communication.

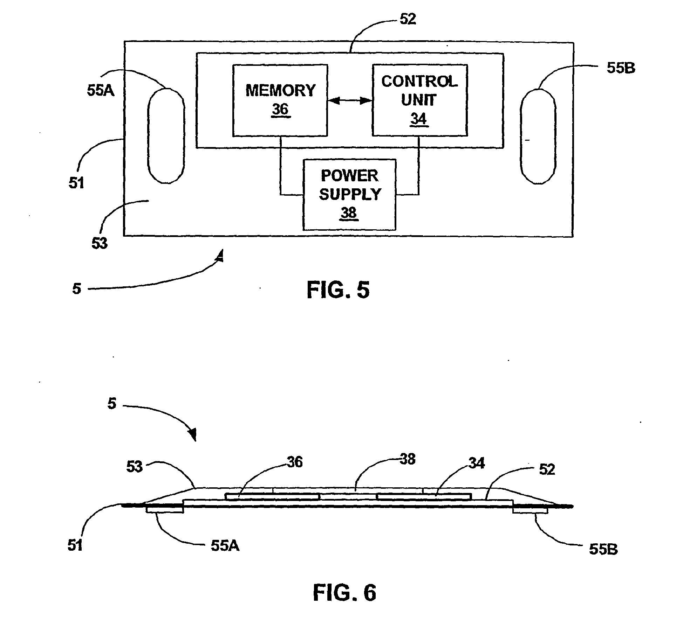

FIGS. 5 and 6 are diagrams illustrating one embodiment of programmer 5 in the form of an adhesive patch. In that case, programmer 5 may include an adhesive strip 51 for attaching programmer 5 to a patients skin. In addition, electrodes 55 may also be used to facilitate the reception of signals though the patients flesh, although the use of electrodes would not be necessary for every embodiment. In other words electrodes 55 may provide an alternative to antenna 52 for the transmission and reception of signals. Accordingly, both electrodes 55 and antenna 52 may be electrically coupled to control unit 34.

Programmer 5 (in this case a patch) is configured to dynamically select different communication modes according to the communication modes presented from medical devices 8. As illustrated, programmer 5 may include an antenna 52, a control unit 34, a memory 36, and a power supply 38, an adhesive strip 51, a protective sheath 53 and electrodes 55.

Antenna 52 may comprise a coplanar dual coil antenna that sends and receives electromagnetic telemetry signals as directed by control unit 34. Alternatively or additionally, electrodes 55 may be used to send and receive the signals. The protective sheath 53 may substantially encapsulate one or more of the components of programmer 5.

As outlined above, programmer 5 (in this case a patch) supports communication according to a plurality of telemetry modes. Control unit 34 compares received signals with stored signatures by accessing memory 36. Then, after identifying an acceptable match between a stored signature and a received telemetry signal, programmer 5 is able to identify the telemetry technique associated with the medical device that sent the signal.

Memory 36 stores the signatures and may also store configuration parameters associated with different communication modes for control unit 34. In addition, memory 36 may include a lookup table (LUT) that maps signatures to communication modes, i.e., by mapping a number associated with a signature to a number associated with an associated communication mode. Thus, upon identifying a signature associated with a received telemetry signal, control unit 34 can access the LUT in memory 36 to select the proper communication mode. Then, control unit 34 can be configured according to the selected communication mode to output telemetry signals that the medical device associated with the received telemetry signal can understand. In addition, control unit 34 can configure itself so that signals sent from the respective device can be properly demodulated and interpreted.

As mentioned above, memory 36 may comprise a removable memory card. Accordingly, memory 36 may be removed from programmer 5, such as via a slot or hole formed in sheath 53. Alternatively, sheath 53 may be pulled back to expose memory 36, allowing memory 36 to be removed or replaced and possibly sent to a physician for analysis.

In still other embodiments, programmer 5 may be embodied in a wrist watch, a belt, a necklace, a pendent, a piece of jewelry, an adhesive patch, a pager, a key fob, an identification (ID) card, a laptop computer, a hand-held computer, or other mechanical configurations. A programmer 5 having dimensions less than approximately 60 millimeters by 90 millimeters by 15 millimeters can be particularly useful for wearable embodiments. In particular, a configuration similar that that illustrated in FIGS. 5 and 6 using the exemplary components listed herein may be used to realize a programmer with a small enough form factor to facilitate different wearable embodiments. Additional components may also be added, such as a magnet or electromagnet used to initiate telemetry for some devices.

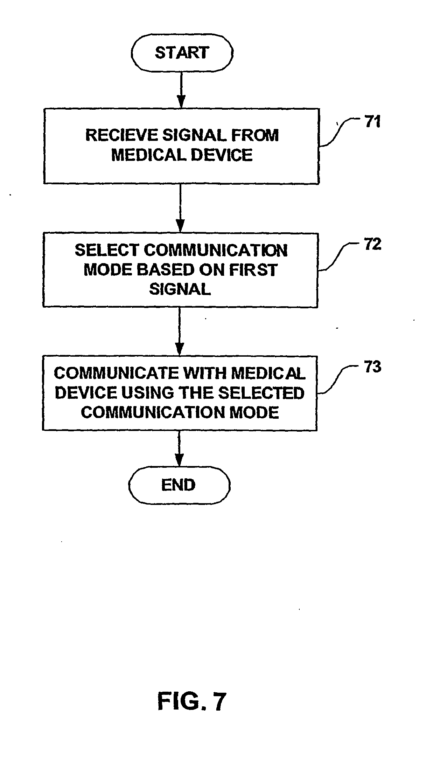

FIG. 7 is a flow diagram illustrating a technique consistent with the principles of the invention. As shown, programmer 5 receives a telemetry signal 12 from a medical device 8 (71). Control unit 24 of programmer 5 selects a communication mode based on the received signal (72). Programmer 5 then communicates with medial device 8 using the selected communication mode (73).
In order to select the proper communication mode based on the received signal (72), control unit 24 of programmer 5 may identify a signature associated with the received signal. More specifically, receiver module 46 conditions a received telemetry signal so that it falls within the dynamic range of DSP 42. DSP 42 samples the conditioned signal and compares the digital sample to various signatures stored in memory 36. For example, DSP 42 may perform a correlation operation to compare a digital sample of a received signal to various signatures stored in memory 36. In particular, the correlation operation may compare the frequencies, phase shifts, pulse widths, or any other variable between the digital sample of the received signal to those of the different signatures. Upon identifying a signature that matches the digital sample of the received signal to within an acceptable degree (which may also be programmable), DSP 42 can configure programmer 5 according to a communication mode associated with the signature. In other words, once the appropriate signature has been identified, DSP 42 can select a communication mode, such as by accessing a LUT in memory 36 that maps signatures to communication modes.

Upon identifying the necessary communication mode for telemetric communication with a respective medical device 8, control unit 34 configures for such communication. For example, DSP 42 may select an appropriate set of bandpass filters and an appropriate demodulator, each of which may be software implemented as part of DSP 42. In addition, in some cases, DSP 42 may send control signals to receiver module 46 and transmitter module 48 to configure those modules 46, 48 for respective reception and transmission consistent with the selected communication mode. In this manner, programmer 5 can be configured for communication according to a first telemetric mode of communication, and then reconfigured for communication according to a second (different) telemetric mode of communication. In some cases, a large number of different communication modes can be supported by programmer 5.

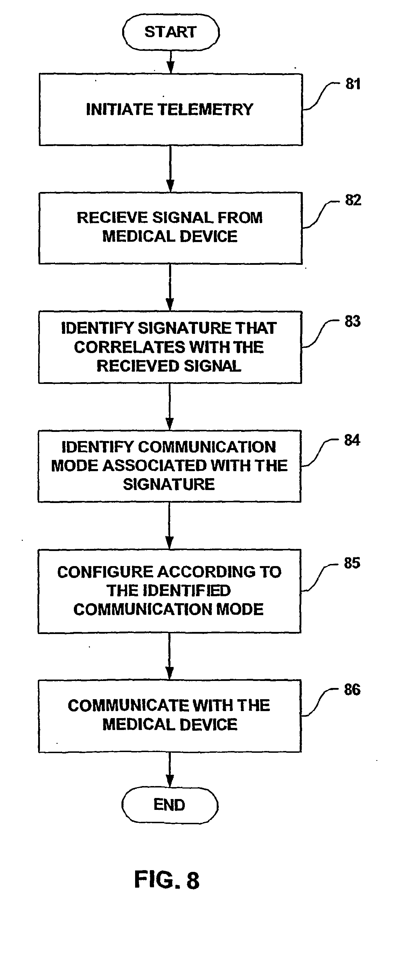

FIG. 8 is another flow diagram illustrating a technique consistent with the principles of the invention. As shown, programmer 5 initiates telemetry with a medical device 8 (81). In most cases, in order to preserve battery life in a medical device, the medical device does not send telemetry signals unless it receives a request for such signals. Accordingly, programmer 5 can be configured to initiate telemetry with medical devices (81) by sending an appropriate request. Moreover, since the initiation required to cause a given medical device to send a telemetry signal may differ with the device, programmer may perform a plurality of initiation techniques so as to cause any device supported by programmer 5 to send a telemetry signal. For some devices, a magnetic field may be used to initiate telemetry, such as by magnetically activating a switch on the respective device to cause the device to send telemetry signals. Accordingly, programmer 5 may include a magnet or an electromagnet that generates the required magnetic field to cause the medical device to send a telemetry signal. For other devices, telemetry from a medical device 8 may begin upon receiving a particular wireless signal that corresponds to a request for telemetry. Accordingly, control unit 24 of programmer 5 may be configured to send one or more different request signals to provoke a response from the medical device. In some cases, control unit 24 may send different request signals over time to provoke responses from different medical devices for which programmer 5 supports telemetry. Thus, if a particular device is in proximity to programmer 5, eventually the appropriate request signal will be sent from programmer 5 to that device.

In any case, once programmer 5 has initiated telemetry with the medical device (81) causing the medical device to send a telemetry signal, programmer 5 receives the signal from the medical device (82). Control unit 24 of programmer 5 identifies a signature stored in memory 26 that correlates with the received signal (83). More specifically, DSP 42 generates a digital sample based on a signal conditioned by receiver module 46, and compares the digital sample to the signatures stored in memory by invoking a correlation operation.

Upon identifying a signature stored in memory 26 that correlates with the received signal (83), control unit 24 identifies a medical device associated with the signature (84). More specifically, DSP 42 accesses a LUT in memory 26 which maps signatures to communication modes, and selects from the LUT, the communication mode associated with the identified signature.

Control unit 24 then configures programmer 5 for communication with the medical device 5 according to the selected communication mode (85). More specifically, DSP 42 selects particular bandpass filter(s) and a demodulator to process received telemetry signals in accordance with the communication mode that is selected. In addition, DSP 42 may send control signals to one or more components included in receiver module 46 or transmitter module 48 to configure the modules to condition received signals and to condition output signals according to the communication mode that is selected. Programmer 5 can then telemetrically communicate with the medical device (86). This telemetric communication may be used for any of a wide variety of desirable communication that can occur between a programmer and a medical device. For example, programmer 5 may telemetrically communicate with the medical device to program a new therapy technique into the medical device. In particular, device 5 may be configured to receive input from a physician or medical personnel specifying a therapy to be performed, and may send a signal to the medical device according to the selected communication mode to direct the medical device to perform the therapy. Alternatively, programmer 5 may telemetrically communicate with the medical device 8 to request stored information corresponding, for example, to diagnostic information, sensed conditions associated with the patient, information relating to therapy delivered to the patient, or any other information collected or identified by the medical device. In that case, programmer 5 may receive the requested information from the medical device in response to the request for stored information sent according to the appropriately selected communication mode. These or other communications may occur between a medical device and programmer 8 once programmer has identified the appropriate communication mode, and configured according to that communication mode consistent with the principles of the invention.

A number of embodiments and features of a programmer have been described. The programmer may take a variety of forms and mechanical configurations in addition to those described herein. Moreover, the techniques described herein may be implemented in a programmer in hardware, software, firmware, or any combination thereof. If implemented in software, invention may be directed to a computer readable medium comprising program code, that when executed, performs one or more of the techniques described herein. For example, the computer readable medium may comprise a random access memory (RAM), SDRAM, FLASH, or possibly a removable memory card as outlined herein. In any case, the memory stores the computer readable instructions that, when executed cause programmer 5 to carry out the techniques described herein. These and other embodiments are within the scope of the following claims.

## Claims

1. A method for providing communication between a programmer (5) and a plurality of different medical devices (8) which communicate with said programmer using respective, different types of telemetry techniques; said method comprising:
providing a programmer having a plurality of possible types of telemetry communication techniques stored in a memory (36) thereof;
receiving at a control unit of said programmer a first signal from a medical device; and
selecting by means of said control unit a type of communication technique from said plurality of possible communication techniques based on the first signal, for communicating with said medical device.

2. The method of claim 1, wherein selecting the communication technique based on the first signal further includes:
identifying a signature that substantially correlates to the first signal; and
selecting a type of communication technique based on the signature.

3. The method of claim 1, further comprising:
generating a second signal that complies with the selected type of communication technique; and
sending the second signal to the medical device.

4. The method of claim 3, wherein the second signal requests information from the medical device, the method further comprising receiving requested information from the medical device in response to the second signal.

5. The method of any preceding claim, wherein selecting the type of communication technique from a plurality of possible types of communication techniques based on the first signal includes:
accessing a lookup table that maps numbers associated with signatures to numbers associated with possible types of communication techniques;
identifying a signature that substantially correlates to the first signal; and
selecting the type of communication technique based on the identified signature using the lookup table.

6. The method of claim 5, further comprising configuring transmit circuitry (46, 48, 32) for communication with the medical device in accordance with the selected type of communication technique.

7. The method of any preceding claim, further comprising initiating telemetry to cause the medical device to send the first signal.

8. The method of claim 7, wherein initiating telemetry includes performing a plurality of possible types of telemetry initiation techniques.

9. A programmer for a plurality of different medical devices which communicate with the programmer using different types of telemetry techniques, said programmer comprising:
memory (36) storing a plurality of possible types of telemetry communication techniques for the programmer; and
a control unit (34) to receive a first signal from a medical device (8) and select one of the types of communication techniques based on the first signal to communication with said medical device.

10. The programmer of claim 9, wherein the control unit selects the type of communication technique based on the first signal by:
identifying a signature that substantially correlates to the first signal; and
selecting a type of communication technique associated with the signature.

11. The programmer of claim 9, the control unit being configured to:
generate a second signal that complies with the selected type of communication technique; and
send the second signal to the medical device.

12. The programmer of claim 11, wherein the second signal requests information from the medical device, wherein the control unit receives requested information from the medical device in response to the second signal.

13. The programmer of any of claims 9 to 12, the control unit being configured to:
receive input specifying a therapy;
generate a second signal that complies with the selected type of communication technique to program the medical device to implement the therapy; and
send the second signal to the medical device.

14. The programmer of any of claims 9 to 13, wherein the memory stores a lookup table that maps numbers associated with the plurality of possible types of communication techniques to numbers associated with a set of signatures, and wherein the control unit selects the communication technique from a plurality of possible types of communication techniques based on the first signal by:
accessing the lookup table;
identifying a signature that substantially correlates to the first signal; and
selecting the type of communication technique based on the identified signature using the lookup table.

15. The programmer of claim 14, wherein the control unit configures for communication with the medical device in compliance with the selected type of communication technique.

16. The programmer of any of claims 9 to 15, the control unit being configured to initiate telemetry to cause the medical device to send the first signal.

17. The programmer of claim 16, wherein the control unit initiates telemetry by performing a plurality of possible telemetry initiation techniques.

18. The programmer of any of claims 9 to 17, wherein the programmer forms at least part of an apparatus selected from the following group: a wrist watch, a belt, a necklace, a piece of jewelry, an adhesive patch, a pager, a key fob, an identification card, a laptop computer, an interrogator, and a hand-held computer.

19. The programmer of any of claims 9 to 18, wherein the memory comprises a removable memory card.

20. The programmer of any of claims 9 to 19, further comprising a patient activator to allow the patient to activate the programmer.

21. The programmer of any of claims 9 to 20, further comprising a feedback device to indicate to a patient that telemetric communication has been initiated.

22. Program code for execution by a processor in a programmer as claimed in any of claims 9 to 21, whereby the program encodes the method of any of claims 1 to 8.

## Patentansprüche

1. Verfahren zum Bereitstellen einer Kommunikation zwischen einer Programmiereinrichtung bzw. einem Programmierer (5) und einer Mehrzahl von verschiedenen medizinischen Einrichtungen (8), die mit dem Programmierer kommunizieren, wobei jeweilige verschiedene Typen von Telemetrie- bzw. Übertragungstechniken verwendet werden, wobei das Verfahren folgende Schritte umfasst:
Bereitstellen eines Programmierers mit einer Mehrzahl von möglichen Typen von Telemetrie-Kommunikationstechniken, die in einem Speicher (36) davon abgelegt sind,
Empfangen bei einer Steuereinheit des Programmierers eines ersten Signals von einer medizinischen Einrichtung, und
Auswählen mittels der Steuereinheit eines Typs einer Kommunikationstechnik von der Mehrzahl von möglichen Kommunikationstechniken basierend auf dem ersten Signal, um mit der medizinischen Einrichtung zu kommunizieren.

2. Verfahren nach Anspruch 1, bei dem der Schritt des Auswählens der Kommunikationstechnik basierend auf dem ersten Signal weiterhin folgende Schritte umfasst:
Identifizieren einer Signatur, die im wesentlichen mit dem ersten Signal korreliert, und
Auswählen eines Typs einer Kommunikationstechnik basierend auf der Signatur.

3. Verfahren nach Anspruch 1, das weiterhin die Schritte umfasst:
Erzeugen eines zweiten Signals, das mit dem ausgewählten Typ einer Kommunikationstechnik übereinstimmt, und
Senden des zweiten Signals zu der medizinischen Einrichtung.

4. Verfahren nach Anspruch 3, bei dem das zweite Signal Informationen von der medizinischen Einrichtung abfragt bzw. anfordert, wobei das Verfahren weiterhin den Schritt des Empfangens angeforderter Informationen von der medizinischen Einrichtung in Reaktion auf das zweite Signal umfasst.

5. Verfahren nach einem der vorstehenden Ansprüche, bei dem der Schritt des Auswählens des Typs einer Kommunikationstechnik von einer Mehrzahl von möglichen Typen von Kommunikationstechniken basierend auf dem ersten Signal die Schritte umfasst:
Zugreifen auf eine Nachschlagetabelle, die Zahlen, die mit Signaturen in Verbindung stehen, auf Zahlen abbildet, die mit möglichen Typen von Kommunikationstechniken in Verbindung stehen,
Identifizieren einer Signatur, die im wesentlichen mit dem ersten Signal korreliert, und
Auswählen des Typs einer Kommunikationstechnik basierend auf der identifizierten Signatur, wobei die Nachschlagetabelle verwendet wird.

6. Verfahren nach Anspruch 5, das weiterhin den Schritt des Konfigurierens eines Übertragungsschaltkreises (46, 48, 32) für eine Kommunikation mit der medizinischen Einrichtung in Übereinstimmung mit dem ausgewählten Typ einer Kommunikationstechnik umfasst.

7. Verfahren nach einem der vorstehenden Ansprüche, das weiterhin den Schritt des Initiierens einer Telemetrie umfasst, um zu bewirken, dass die medizinische Einrichtung das erste Signal sendet.

8. Verfahren nach Anspruch 7, bei dem der Schritt des Initiierens einer Telemetrie den Schritt des Durchführens einer Mehrzahl von möglichen Typen von Telemetrie-Initiierungstechniken umfasst.

9. Programmierer für eine Mehrzahl von verschiedenen medizinischen Einrichtungen, die mit dem Programmierer kommunizieren, wobei verschiedene Typen von Telemetrie-Techniken verwendet werden, wobei der Programmierer aufweist:
einen Speicher (36) zum Speichern einer Mehrzahl von möglichen Typen von Telemetrie-Kommunikationstechniken für den Programmierer, und
eine Steuereinheit (34), um ein erstes Signal von einer medizinischen Einrichtung (8) zu empfangen und einen der Typen von Kommunikationstechniken basierend auf dem ersten Signal für eine Kommunikation mit der medizinischen Einrichtung auszuwählen.

10. Programmierer nach Anspruch 9, bei dem die Steuereinheit den Typ einer Kommunikationstechnik basierend auf dem ersten Signal durch folgende Schritte auswählt:
Identifizieren einer Signatur, die im wesentlichen mit dem ersten Signal korreliert, und
Auswählen eines Typs einer Kommunikationstechnik, die mit der Signatur in Verbindung steht.

11. Programmierer nach Anspruch 9, wobei die Steuereinheit konfiguriert ist, um:
ein zweites Signal zu erzeugen, das mit dem ausgewählten Typ einer Kommunikationstechnik übereinstimmt, und
das zweite Signal zu der medizinischen Einrichtung zu senden.

12. Programmierer nach Anspruch 11, bei dem das zweite Signal Informationen von der medizinischen Einrichtung anfordert, wobei die Steuereinheit angeforderte Informationen von der medizinischen Einrichtung in Reaktion auf das zweite Signal empfängt.

13. Programmierer nach einem der Ansprüche 9 bis 12, wobei die Steuereinheit konfiguriert ist, um:
eine Eingabe zu empfangen, die eine Therapie spezifiziert,
ein zweites Signal zu erzeugen, das mit dem ausgewählten Typ eine Kommunikationstechnik übereinstimmt, um die medizinische Einrichtung zu programmieren, um die Therapie zu implementieren, und
das zweite Signal zu der medizinischen Einrichtung zu senden.

14. Programmierer nach einem der Ansprüche 9 bis 13, bei dem der Speicher eine Nachschlagetabelle speichert, die Zahlen, die mit der Mehrzahl von möglichen Typen von Kommunikationstechniken in Verbindung stehen, auf Zahlen, die mit einem Satz an Signaturen in Verbindung stehen, abbildet, und wobei die Steuereinheit die Kommunikationstechnik von einer Mehrzahl von möglichen Typen von Kommunikationstechniken basierend auf dem ersten Signal auswählt, indem:
auf die Nachschlagetabelle zugegriffen wird,
eine Signatur identifiziert wird, die im wesentlichen mit dem ersten Signal korreliert, und
der Typ einer Kommunikationstechnik basierend auf der identifizierten Signatur ausgewählt wird, wobei die Nachschlagetabelle verwendet wird.

15. Programmierer nach Anspruch 14, bei dem die Steuereinheit für eine Kommunikation mit der medizinischen Einrichtung in Übereinstimmung mit dem ausgewählten Typ einer Kommunikationstechnik konfiguriert.

16. Programmierer nach einem der Ansprüche 9 bis 15, wobei die Steuereinheit konfiguriert ist, um eine Telemetrie zu initiieren, um zu bewirken, dass die medizinische Einrichtung das erste Signal sendet.

17. Programmierer nach Anspruch 16, bei dem die Steuereinheit eine Telemetrie durch Ausführen einer Mehrzahl von möglichen Telemetrie-Initiierungstechniken initiiert.

18. Programmierer nach einem der Ansprüche 9 bis 17, bei dem der Programmierer zumindest einen Teil einer Vorrichtung bildet, die aus der folgenden Gruppe ausgewählt ist:
eine Armbanduhr, ein Band, ein Halsband, ein Juwelenstück, ein Klebeaufnäher, ein Pager bzw. Taschenpiepser, ein Schlüsselanhänger, eine Identifikationskarte, ein Laptopcomputer, eine Abfrageeinrichtung und ein tragbarer Computer.

19. Programmierer nach einem der Ansprüche 9 bis 18, bei dem der Speicher eine abnehmbare bzw. austauschbare Speicherkarte umfasst.

20. Programmierer nach einem der Ansprüche 9 bis 19, der weiterhin einen Patientenaktivator umfasst, um dem Patienten zu ermöglichen, den Programmierer zu aktivieren.

21. Programmierer nach einem der Ansprüche 9 bis 20, der weiterhin eine Rückkopplungseinrichtung aufweist, um einem Patienten anzuzeigen, dass eine Telemetrie-Kommunikation initiiert wurde.

22. Programmiercode zum Ausführen durch einen Prozessor in einen Programmierer nach einem der Ansprüche 9 bis 21, wodurch das Programm das Verfahren nach einem der Ansprüche 1 bis 8 codiert.

## Revendications

1. Procédé pour réaliser une communication entre un programmateur (5) et une pluralité de différents dispositifs médicaux (8) qui communiquent avec ledit programmateur en utilisant différents types de techniques de télémétrie respectives, ledit procédé comportant les étapes consistant à :
fournir un programmateur ayant une pluralité de types possibles de techniques de communication de télémétrie mémorisées dans une mémoire (36) de celui-ci ;
recevoir dans une unité de commande dudit programmateur un premier signal provenant d'un dispositif médical ; et
sélectionner par l'intermédiaire de ladite unité de commande un type de technique de communication parmi ladite pluralité de possibles techniques de communication d'après le premier signal, pour communiquer avec ledit dispositif médical.

2. Procédé selon la revendication 1, dans lequel la sélection de la technique de communication sur la base du premier signal inclut de plus :
l'identification d'une signature qui est sensiblement corrélée au premier signal ; et
la sélection d'un type de technique de communication d'après la signature.

3. Procédé selon la revendication 1, comportant de plus :
la génération d'un second signal qui s'accorde avec le type sélectionné de technique de communication ; et
l'envoi du second signal au dispositif médical.

4. Procédé selon la revendication 3, dans lequel le second signal demande des informations dans le dispositif médical, le procédé comportant de plus la réception d'informations demandées depuis le dispositif médical en réponse au second signal.

5. Procédé selon l'une quelconque des revendications précédentes, dans lequel la sélection du type de technique de communication parmi une pluralité de possibles types de techniques de communication sur la base du premier signal inclut :
l'accès à une table de consultation qui mappe des numéros associés à des signatures en des numéros associés à de possibles types de techniques de communication ;
l'identification d'une signature qui est sensiblement corrélée au premier signal ; et
la sélection du type de technique de communication d'après la signature identifiée en utilisant la table de consultation.

6. Procédé selon la revendication 5, comportant de plus la configuration d'un circuit de transmission (46, 48, 32) pour communiquer avec le dispositif médical conformément au type de technique de communication sélectionné.

7. Procédé selon l'une quelconque des revendications précédentes, comportant de plus le lancement d'une télémétrie pour amener le dispositif médical à envoyer le premier signal.

8. Procédé selon la revendication 7, dans lequel le lancement de télémétrie inclut l'exécution d'une pluralité de types possibles de techniques de lancement de télémétrie.

9. Programmateur pour une pluralité de différents dispositifs médicaux qui communiquent avec le programmateur en utilisant différents types de techniques de télémétrie, ledit programmateur comportant:
une mémoire (36) mémorisant une pluralité de types possibles de techniques de communication de télémétrie pour le programmateur ; et
une unité de commande (34) pour recevoir un premier signal depuis un dispositif médical (8) et sélectionner l'un des types de techniques de communication d'après le premier signal pour communiquer avec ledit dispositif médical.

10. Programmateur selon la revendication 9, dans lequel l'unité de commande sélectionne le type de technique de communication d'après le premier signal en :
identifiant une signature qui est sensiblement corrélée au premier signal ; et
sélectionnant un type de technique de communication associé à la signature.

11. Programmateur selon la revendication 9, l'unité de commande étant configurée pour :
générer un second signal qui s'accorde avec le type de technique de communication sélectionné ; et
envoyer le second signal au dispositif médical.

12. Programmateur selon la revendication 11, dans lequel le second signal demande des informations dans le dispositif médical, dans lequel l'unité de commande reçoit des informations demandées à partir du dispositif médical en réponse au second signal.

13. Programmateur selon l'une quelconque des revendications 9 à 12, l'unité de commande étant configurée pour :
recevoir une entrée spécifiant une thérapie;
générer un second signal qui s'accorde avec le type de technique de communication sélectionné pour programmer le dispositif médical de manière à mettre en oeuvre la thérapie ; et
envoyer le second signal au dispositif médical.

14. Programmateur selon l'une quelconque des revendications 9 à 13, dans lequel la mémoire mémorise une table de consultation qui mappe des numéros associés à la pluralité de types possibles de techniques de communication en des numéros associés à un ensemble de signatures, et dans lequel l'unité de commande sélectionne la technique de communication parmi une pluralité de types possibles de techniques de communication sur la base du premier signal en :
accédant à la table de consultation ;
identifiant une signature qui est sensiblement corrélée au premier signal ; et
sélectionnant le type de technique de communication d'après la signature identifiée en utilisant la table de consultation.

15. Programmateur selon la revendication 14, dans lequel l'unité de commande est configurée pour communiquer avec le dispositif médical en conformité avec le type de technique de communication sélectionné.

16. Programmateur selon l'une quelconque des revendications 9 à 15, l'unité de commande étant configurée pour lancer une télémétrie pour amener le dispositif médical à envoyer le premier signal.

17. Programmateur selon la revendication 16, dans lequel l'unité de commande lance une télémétrie en effectuant une pluralité de techniques de lancement de télémétrie possibles.

18. Programmateur selon l'une quelconque des revendications 9 à 17, dans lequel le programmateur forme au moins une partie d'un dispositif sélectionné parmi le groupe suivant : une montre-bracelet, une ceinture, un collier, une partie de bijou, un timbre adhésif, un téléavertisseur, un porte-clé, une carte d'identification, un ordinateur portable, un interrogateur, et un ordinateur portatif.

19. Programmateur selon l'une quelconque des revendications 9 à 18, dans lequel la mémoire comporte une carte mémoire amovible.

20. Programmateur selon l'une quelconque des revendications 9 à 19, comportant de plus un activateur de patient pour permettre au patient d'activer le programmateur.

21. Programmateur selon l'une quelconque des revendications 9 à 20, comportant de plus un dispositif de rétroaction pour indiquer à un patient qu'une communication télémétrique a été lancée.

22. Code de programme destiné à être exécuté par un processeur dans un programmateur selon l'une quelconque des revendications 9 à 21, de sorte que le programme code le procédé selon l'une quelconque des revendications 1 à 8.
